# DEMANDE DE BREVET EUROPEEN

(11) **EP 1 174 121 A1**
(43) Date de publication de la demande: **23.01.2002**
(21) Numéro de dépôt: 01401690.1
(22) Date de dépôt: 26.06.2001
(51) Int. Cl.: A61K 7/48

(54) **Composition comprenant un extrait de sophora japonica et utilisation en cosmétique**

(30) Priorité: 18.07.2000 FR 0009403
(71) Demandeur: L'OREAL, 75008 Paris (FR)
(72) Inventeur: Liviero, Christel, 75008 Paris (FR); Pelletier, Pascale, 92160 Antony (FR); Breton, Lionel, 78000 Versailles (FR)
(74) Mandataire: Galup, Cédric Olivier Nicolas

(57) **Abrégé**

L'invention concerne l'utilisation d'au moins un extrait d'au moins un végétal de l'espèce *Sophora japonica*, dans une composition ou pour la préparation d'une composition, l'extrait ou la composition étant destinés à favoriser la desquamation de la peau et/ou stimuler le renouvellement épidermique et/ou lutter contre le vieillissement de la peau. Elle se rapporte aussi à des compositions pour favoriser la desquamation de la peau et/ou stimuler le renouvellement épidermique et donc lutter contre le vieillissement cutané intrinsèque et/ou extrinsèque, ainsi qu'à un procédé de traitement non thérapeutique de la peau destiné à favoriser la desquamation et/ou lutter contre le vieillissement de la peau.

## Description

L'invention concerne l'utilisation d'au moins un extrait d'au moins un végétal de l'espèce *Sophora japonica*, dans une composition ou pour la préparation d'une composition, l'extrait ou la composition étant destinés à favoriser la desquamation de la peau et/ou stimuler le renouvellement épidermique et/ou lutter contre le vieillissement de la peau. Elle se rapporte aussi à des compositions pour favoriser la desquamation de la peau et/ou stimuler le renouvellement épidermique et donc lutter contre le vieillissement cutané intrinsèque et/ou extrinsèque, ainsi qu'à un procédé de traitement non thérapeutique de la peau destiné à favoriser la desquamation et/ou lutter contre le vieillissement de la peau.

La desquamation est un phénomène naturel lié au fait que l'épiderme, qui constitue la couche supérieure de la peau, est en constante régénération. L'épiderme est constitué de plusieurs assises de cellules, dont la plus profonde est l'assise basale constituée de cellules indifférenciées. Au cours du temps, ces cellules vont différencier et migrer vers la surface de l'épiderme en constituant les différentes assises de celui-ci, jusqu'à former à la surface de l'épiderme les cornéocytes qui sont des cellules mortes qui s'éliminent par desquamation. Cette perte en surface est compensée par la migration de cellules de l'assise basale vers la surface de l'épiderme. Il s'agit du renouvellement perpétuel de la peau. Une élimination forcée de la couche cornée accélère le renouvellement et permet de lutter contre le vieillissement.
Dans le même temps ces cellules poursuivent leur différenciation dont le dernier stade est le cornéocyte. Il s'agit en fait de cellules mortes qui constituent la dernière couche de l'épiderme, c'est à dire la couche la plus externe encore appelée stratum corneum.

Le vieillissement cutané résultant de facteurs intrinsèques ou extrinsèques se traduit par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté ou par l'apparition de télangiectasies.

Certains de ces signes du vieillissement sont plus particulièrement liés au vieillissement intrinsèque ou physiologique, c'est-à-dire au vieillissement « normal » lié à l'âge ou chronobiologique, alors que d'autres sont plus spécifiques du vieillissement extrinsèque, c'est-à-dire du vieillissement provoqué d'une manière générale par l'environnement ; il s'agit plus particulièrement du photovieillissement dû à l'exposition au soleil, à la lumière ou à tout autre rayonnement.

L'invention s'intéresse au vieillissement intrinsèque ou physiologique ainsi qu'au vieillissement extrinsèque.

Les changements de la peau dus au vieillissement intrinsèque sont la conséquence d'une sénescence génétiquement programmée où interviennent des facteurs endogènes. Ce vieillissement intrinsèque provoque notamment un ralentissement du renouvellement des cellules de la peau, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique.

Au contraire, le vieillissement extrinsèque entraîne des altérations cliniques telles que des rides épaisses et la formation d'une peau molle et tannée, et des changements histopathologiques tels qu'une excessive accumulation de matière élastique dans le derme supérieur et une dégénérescence des fibres de collagène.

On connaît dans l'art antérieur divers agents destinés à lutter contre le vieillissement cutané.

Ainsi, le brevet US-A-4603146 décrit l'emploi d'acide rétinoïque et de ses dérivés dans des compositions cosmétiques, en vue de lutter contre le vieillissement cutané.
Par ailleurs, de nombreux brevets et publications (voir par exemple la demande EP-A-413528) ainsi que de nombreuses compositions cosmétiques du commerce enseignent l'emploi des α-hydroxyacides comme l'acide lactique, l'acide glycolique ou encore l'acide citrique pour traiter le vieillissement cutané.

On connaît enfin les β-hydroxyacides et plus spécialement l'acide salicylique ainsi que ses dérivés pour leur propriétés desquamantes (voir les documents WO-A-93/10756 et US-A-4 767 750).

Tous ces composés ont une action contre le vieillissement de la peau en favorisant la desquamation, c'est-à-dire l'élimination des cellules « mortes » situées à la surface de la couche cornée de l'épiderme. Cette propriété "desquamante" est aussi appelée, souvent à tort, propriété kératolytique.

Mais les composés de l'art antérieur présentent également des effets secondaires, qui consistent en des picotements, des tiraillements, des échauffements et des rougeurs désagréables pour l'utilisateur.

On constate donc que subsiste le besoin d'agents antivieillissement ayant une action au moins aussi efficace que celle des composés de l'art antérieur, mais ne présentant pas leurs inconvénients.

L'invention a pour but de pallier les inconvénients des solutions de l'art antérieur et de proposer une composition favorisant la desquamation de la peau et/ou stimulant le renouvellement épidermique, dont l'utilisation n'entraînerait pas de picotements, de tiraillements, d'échauffements ou de rougeurs désagréables pour l'utilisateur.

La demanderesse a trouvé de manière inattendue que l'application d'au moins un extrait d'au moins un végétal de l'espèce *Sophora japonica* permet de favoriser la desquamation de la peau et/ou stimuler le renouvellement épidermique donc de lutter contre le vieillissement.

Dans les publications de l'art antérieur les extraits d'au moins un végétal de l'espèce *Sophora japonica* sont connus pour leur propriété tinctoriale particulièrement dans la teinture de la soie mais aussi dans la pharmacie du fait de leur teneur en rutoside (Jean Bruneton, phytochimie, plantes médicinales 2^{ème} édition, technique et documentation- lavoisier, p 281,1993).
Des propriétés anti-bactériennes ont aussi été décrites (Kimura M, Yamada H ; « Interaction in the antibacterial activity of flavonoids from sophra japonica L. to Propionibacterium »; *Yakugaku Zasshi ;* 104 (4) :340-6 ; 1984).

Des propriétés antihémorrhagiques sont également mentionnées dans l'art antérieur (Ishida H, Umino T. : « studies on antihemorrhagic substances in herbs classified as hemostatics in chinese medecine. VI. On the antihemorrhagic principle in *Sophora japonica* L. », *chem pharm Bull;* 35(2) :857-60; 1987).

A la connaissance de la demanderesse, il n'a jamais été décrit dans l'art antérieur l'utilisation d'au moins un extrait d'au moins un végétal de l'espèce *Sophora japonica* pour favoriser la desquamation de la peau et/ou stimuler le renouvellement épidermique et donc lutter contre le vieillissement cutané intrinsèque et/ou extrinsèque.

L'invention a donc pour premier objet l'utilisation d'au moins un extrait d'au moins un végétal de l'espèce *Sophora japonica* dans une composition ou pour la préparation d'une composition, l'extrait ou la composition étant destinés à favoriser la desquamation de la peau et/ou stimuler le renouvellement épidermique et donc lutter contre le vieillissement cutané intrinsèque et/ou extrinsèque.

L'espèce *Sophora japonica* appartient au genre végétal Sophora qui lui même appartient à la famille des Légumineuses. Communément appelé l'arbre des pagodes, il est originaire du centre et du nord de la chine.

Bien entendu, l'extrait peut être préparé à partir d'au moins l'une quelconque des nombreuses variétés associées à chacune des espèces végétales appartenant au genre Sophora. On comprend donc que dans le texte le terme *Sophora japonica* doit s'entendre comme désignant l'une quelconque des nombreuses variétés végétales associées à chacune des espèces végétales appartenant au genre Sophora et particulièrement comme désignant l'espèce *Sophora japonica*.

L'extrait de végétal de l'espèce *Sophora japonica* peut être tout extrait préparé à partir de tout matériel végétal issu d'au moins un végétal du genre Sophora.

Ainsi, le végétal de l'espèce *Sophora japonica* utilisé selon l'invention peut être obtenu à partir de matériel végétal issu de plante entière ou de parties de plante comme les feuilles, les tiges, les fleurs, les pétales, les graines, les racines ou encore des cellules dédifférenciées.

Par cellules végétales dédifférenciées, on entend toute cellule végétale ne présentant aucun des caractères d'une spécialisation particulière et capable de vivre par elle-même et non en dépendance avec d'autres cellules.

Préférentiellement selon l'invention on utilise un extrait préparé à partir de feuilles vertes.

L'extrait d'au moins un végétal de l'espèce *Sophora japonica* peut être tout extrait préparé à partir de tout matériel végétal issu d'au moins un végétal du de l'espèce *Sophora japonica* cultivé *in vivo* ou issu de culture *in vitro*.

Par culture *in vivo* on entend toute culture de type classique c'est à dire en sol à l'air libre ou en serre, ou encore hors sol.

Par culture *in vitro,* on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo*.

Préférentiellement selon l'invention on utilise un végétal issu de culture *in vivo*.

Toute méthode d'extraction connue de l'homme du métier peut être utilisée pour préparer l'extrait contenu dans la composition selon l'invention.

On peut en particulier citer les extraits aqueux, alcooliques ou utilisant un solvant organique.
Par solvant aqueux on entend tout solvant constitué totalement ou pour part d'eau. On peut citer ainsi l'eau elle-même, les solvants hydroalcooliques en toute proportion ou encore les solvants constitués d'eau et d'un composé comme le propylène glycol en toute proportion.

Parmi les solvants alcooliques on peut citer notamment l'éthanol.

On peut également utiliser un extrait préparé par la méthode décrite dans la demande de brevet français n° 95-02379 déposée par la demanderesse.
Ainsi, dans une première étape on broie le matériel végétal dans une solution aqueuse à froid, dans une deuxième étape les particules en suspension sont éliminées de la solution aqueuse issue de la première étape, et dans une troisième étape on stérilise la solution aqueuse issue de la deuxième étape. Cette solution aqueuse correspond à l'extrait.
D'autre part, la première étape peut avantageusement être remplacée par une opération de congélation simple des tissus végétaux (par exemple à -20°C), suivie d'une extraction aqueuse reprenant les deuxièmes et troisième étapes ci-dessus décrites.

Quel que soit le mode de préparation utilisé selon l'invention des étapes subséquentes visant à favoriser la conservation et/ou la stabilisation peuvent être ajoutées sans pour cela modifier la nature même de l'extrait. Ainsi, par exemple l'extrait obtenu peut être lyophilisé par toutes méthodes classiques de lyophilisation. On obtient ainsi une poudre qui peut être utilisée directement ou bien mélanger dans un solvant approprié avant utilisation.

On peut encore citer des extraits commerciaux tels que l'extrait sec de végétal de l'espèce *Sophora japonica* vendu par la société Solabia sous le nom commercial de Sophorine®, préférentiellement utilisé selon l'invention.

Selon un autre aspect, l'invention à pour objet une composition pour favoriser la desquamation de la peau et/ou stimuler le renouvellement épidermique et donc lutter contre le vieillissement cutané intrinsèque et/ou extrinsèque comprenant au moins un extrait d'au moins un végétal de l'espèce *Sophora japonica*.

La quantité d'extrait d'au moins un végétal de l'espèce *Sophora japonica* utilisable selon l'invention, dépend bien évidemment de l'effet recherché et doit être en une quantité efficace pour favoriser la desquamation de la peau et/ou stimuler le renouvellement épidermique et donc lutter contre le vieillissement cutané intrinsèque et/ou extrinsèque.

A titre d'exemple la quantité d'extrait d'au moins un végétal de l'espèce *Sophora japonica* utilisable selon l'invention peut aller par exemple de 0.001% à 20% et de préférence de 0.01% à 10% du poids total de la composition.

Selon encore un autre aspect, l'invention a pour objet un procédé de traitement non thérapeutique pour favoriser la desquamation de la peau et/ou stimuler le renouvellement épidermique et donc lutter contre le vieillissement cutané intrinsèque et/ou extrinsèque, caractérisé en ce qu'on applique sur la peau une composition cosmétique comprenant au moins un extrait d'au moins un végétal de l'espèce *Sophora japonica*.

Ladite composition peut se présenter sous toutes les formes galéniques connues comme par exemple sous la forme d'une émulsion, notamment huile-dans-eau ou eau-dans-huile, voire sous la forme d'une émulsion multiple.
Elle peut également se présenter sous forme d'une solution aqueuse, éventuellement gélifiée, ou sous forme d'une lotion, par exemple biphasée, d'une crème, d'un lait, voire d'une mousse.

Les compositions de l'invention peuvent être ingérées, injectées ou appliquées sur la peau (sur toute zone cutanée du corps), les cheveux, les ongles ou les muqueuses (buccale, jugale, gingivale, génitale, conjonctive). Selon le mode d'administration, les compositions selon l'invention peuvent se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.
La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés, de sirops ou de comprimés.

Les quantités des différents constituants des compositions selon l'invention sont celles classiquement utilisées dans les domaines considérés.

Les compositions selon l'invention peuvent également consister en des préparations solides constituant des savons ou des pains de nettoyage.

Les compositions peuvent aussi être conditionnées sous forme d'une composition pour aérosol comprenant également un agent propulseur sous pression.

Lorsque la composition est une émulsion, la proportion de la phase grasse peut aller de 5% à 80% en poids, et de préférence de 5% à 50% en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

Lorsque la composition est une solution ou un gel huileux, la phase grasse peut représenter plus de 90% du poids total de la composition.

Les compositions de l'invention peuvent être à usage cosmétique ou pharmaceutique. Préférentiellement, les compositions de l'invention sont des compositions à usage cosmétique.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01% à 10% du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).
Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un composé de formule (I) à d'autres agents actifs. Parmi ces agents actifs, on peut citer à titre d'exemple :
- les agents améliorant la repousse et/ou sur le ralentissement de la chute des cheveux, et ayant déjà été décrits pour cette activité comme par exemple les esters d'acide nicotinique, dont notamment le nicotinate de tocophérol, le nicotinate de benzyle et les nicotinates d'alkyles en C₁-C₆ comme les nicotinates de méthyle ou d'hexyle, les dérivés de pyrimidine, comme le 2,4-diamino 6-piperidinopyrimidine 3-oxyde ou "Minoxidil" décrit dans les brevets US 4 139 619 et US 4 596 812, les agents favorisant la repousse des cheveux comme ceux décrits par la demanderesse dans la demande de brevet européen publiée sous le numéro 0648488 ;
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxyde dismutases, certains chélatants de métaux ou l'acide ascorbique et ses esters ;
- les anti-séborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits d'origine végétale, marine ou bactérienne.
   A la liste ci-dessus, d'autres composés peuvent également être rajoutés, à savoir par exemple le Diazoxyde, la Spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétraénoïque et eicosatriénoïque ou leurs esters et amides, la vitamine D et ses dérivés, des extraits d'origine végétale ou bactérienne.
   Ainsi, selon un mode particulier, la composition selon l'invention comprend également au moins un agent choisi parmi les agents antibactériens, les antiparasitaires, les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents diminuant la différenciation et/ou la prolifération et/ou la pigmentation cutanée, les extraits d'origine végétale, marine ou bactérienne.
   La composition pharmaceutique selon l'invention peut être administrée par voie parentérale, entérale ou encore par voie topique. De préférence, la composition pharmaceutique est administrée par voie topique.
   L'invention est illustrée plus en détail dans les exemples suivants.

### Exemple 1 : Activité d'un extrait de Sophora iaponica dans le modèle de détachement cellulaire in vitro chez l'homme.

### Extrait végétal :

extrait sec de végétal de l'espèce *Sophora japonica* vendu par la société Solabia sous le nom commercial de Sophorine®.

### Peau et montage :

La peau utilisée provenait d'une plastie abdominale. La peau a été posée à plat sur une plaque de verre, recouverte d'une plaque 96 puits sans fond, puis clampée de façon à délimiter 96 puits étanches. Pour éviter le dessèchement, toute l'expérience a eu lieu avec la peau immergée dans un bain de tampon phosphate salin (PBS).
Le produit a été préparé par dilution dans du PBS pour obtenir les concentrations : 1 %, 0,5% et 0,1 %.
Cinquante microlitres de chaque dilution du produit ont été distribués sur la peau dans les puits étanches; l'incubation a été réalisée pendant 18 h, à 20°C. Les puits témoins ont reçu 50 microlitres de PBS. Les traitements ont été réalisés en triplicata. Après un doux et reproductible pipetage, le contenu des puits a été prélevé et déposé dans une autre plaque. Les puits ont été lavés et les solutions ont été cumulées aux 50 microlitres prélevés.

### Visualisation et auantification relative des cornéocytes libérés :

Après dilution, une fraction (20 µl) de chaque échantillon (contenant les éventuels cornéocytes) a été transférée sur nitrocellulose ( Hybond ECL, amersham), à l'aide d'une matrice « Slot Blot » ( Milliblot, Millipore) permettant une quantification densitométrique ultérieure. La membrane est ensuite saturée pendant une nuit, à 4°C, en PBS/0.05% Tween 20 (PBST) contenant 1% de lait écrémé.
Après lavage en PBST, les structures dérivés des cornéocytes ont été marquées par un anticorps polyclonal anti-cornéocyte total (1h, 20°C) puis par un conjugué péroxydase - anticorps anti-immunoglobuline de lapin (Tebu ; 1h, 20°C). La peroxydase fixée a été révélée par chimioluminescence (enhance chemiluminescence, ECL, Amersham) sur film Kodak MP.

La saisie des images a été réalisée sur Gel print 2000i (Biophotonics Corp.). Les analyses densitométriques ont été obtenues à l'aide du logiciel One-D-Scan (Scanalytics).

### Traitement des données :

les données brutes ont été transférées et traitées sous le logiciel PRISM® (Graph Pad Software). Les comparaisons intergroupes ont été réalisées par analyse de variance (ANOVA), à l'aide du test de comparaison multiple de DUNNETT.

### Résultats

| | Témoin | Miel d'acacia : 5% | E : 0.1% | E : 0.5% | E : 1% |
|---|---|---|---|---|---|
| Activité en % | 100 | 427 | 254 | 246 | 409 |
| | | P<0.01 | p<0.05 | P<0.05 | P<0.01 |
| E : extrait sec de végétal de l'espèce *Sophora japonica* vendu par la société Solabia sous le nom commercial de Sophorine® | | | | | |

L'activité de l'extrait de *Sophora japonica* sur le détachement cellulaire est nette, elle est équivalente à celle de la référence, le miel d'acacia.
En conclusion, les résultats présentés ci-dessus confirment bien le rôle de l'extrait de *Sophora japonica* dans la régulation du détachement des kératinocytes différenciés.

### Exemple 2 : Exemples de formulations illustrant l'invention. Ces compositions ont été obtenues par simple mélange des différents composants.

### Composition 1 : Lait pour le visage

- Sophorine® 1,0 %
- 1,2-di-(10-hydroxy-dec-2-ènoate)
   de 3-[(2-méthoxy)-éthoxy-méthoxy]-propyle 1,0 %
- Monostéarate de glycéryle, stéarate de polyéthylène glycol (100 OE) 3,0 %
- Polymère carboxyvinylique 0,4 %
- Alcool stéarylique 0,7 %
- Protéines de soja 3,0 %
- NaOH 0,4 %
- Conservateur qs
- Eau qsp 100 %

Cette composition se présente sous forme d'un lait pour le visage, ayant de bonnes propriétés cosmétiques, et étant doux et confortable à utiliser.
Le pH de la composition est d'environ 5,5.

### Composition 2 : Lotion

- Sophorine® 0,5 %
- Palmitate d'éthyl-2 hexyle 10,0 %
- Cyclopentadiméthylsiloxane 20,0 %
- Butylène glycol 5,0 %
- Conservateur qs
- Eau qsp 100 %

Cette lotion, qui ne contient pas de tensioactif, favorise la desquamation de la peau.

### Composition 3 : Lait

- Palmitate d'octyle 35,0 %
- Glycérine 2,0 %
- Sophorine® 1,5 %
- Polymère réticulé acrylates C10-C30/alkylacrylates 0,1 %
- Triéthanolamine 0,1 %
- Acides aminés de blé 1,0 %
- Conservateur qs
- Eau qsp 100%

Le lait obtenu, qui ne contient pas de tensioactif, possède de bonnes propriétés cosmétiques.

### Composition 4 : Gel pour le visage

- Glycérine 10,00 %
- Sophorine® 0,05 %
- Cocoamphodiacétate de disodium 1,00 %
- Conservateur qs
- Eau qsp 100 %

Le gel obtenu possède de bonnes propriétés cosmétiques.

### Composition 5 : Gel nettoyant à l'eau

- Butylène glycol 7,0 %
- Sarcosinate de lauroyl sodium 4,0 %
- Sophorine® 0,1 %
- Triéthanolamine 0,8 %
- Carbomer 0,5 %
- Conservateur qs
- Eau qsp 100 %

Le gel obtenu possède de bonnes propriétés cosmétiques.

## Revendications

1. Utilisation d'au moins un extrait d'au moins un végétal de l'espèce *Sophora japonica* dans une composition ou pour la préparation d'une composition, l'extrait ou la composition étant destinés à favoriser la desquamation de la peau.

2. Utilisation d'au moins un extrait d'au moins un végétal de l'espèce *Sophora japonica* dans une composition ou pour la préparation d'une composition, l'extrait ou la composition étant destinés à stimuler le renouvellement épidermique.

3. Utilisation d'au moins un extrait d'au moins un végétal de l'espèce *Sophora japonica* dans une composition ou pour la préparation d'une composition, l'extrait ou la composition étant destinés à lutter contre le vieillissement cutané intrinsèque et/ou extrinsèque.

4. Utilisation selon le revendication précédente, **caractérisée par le fait que** l'extrait est utilisé en une quantité allant de 0.001% à 20% du poids total de la composition.

5. Utilisation selon l'une quelconque des revendications précédentes, **caractérisée par le fait que** l'extrait est utilisé en une quantité allant 0.01% à 10% du poids total de la composition.

6. Utilisation selon l'une quelconque des revendications précédentes, dans une composition se présentant sous la forme d'une émulsion, d'une solution aqueuse, éventuellement gélifiée, d'une lotion notamment biphasée, d'une crème, d'un lait, d'une mousse.

7. Composition pour favoriser la desquamation de la peau et/ou stimuler le renouvellement épidermique et donc lutter contre le vieillissement cutané intrinsèque et/ou extrinsèque, **caractérisée en ce qu'**elle comprend au moins un extrait d'au moins un végétal de l'espèce *Sophora japonica*.

8. Procédé de traitement non thérapeutique pour favoriser la desquamation de la peau et/ou stimuler le renouvellement épidermique et donc lutter contre le vieillissement cutané intrinsèque et/ou extrinsèque, **caractérisé en ce qu'**on applique sur la peau une composition cosmétique telle que définie dans la revendication 7.
